# EUROPEAN PATENT APPLICATION

(11) **EP 3 939 624 A1**
(43) Date of publication of application: **19.01.2022**
(21) Application number: 20186436.0
(22) Date of filing: 17.07.2020
(51) Int. Cl.: A61L 27/36, A61L 27/22, A61L 27/50, A61L 27/52, A61B 17/08, A61F 13/00, A61F 13/62, A44B 18/00, A61B 17/00

(54) **MEDICAL DEVICE AND METHOD OF FABRICATION THEREOF**

(71) Applicant: CSEM Centre Suisse d'Electronique et de Microtechnique SA - Recherche et Développement, 2002 Neuchâtel (CH); Universität Bern, 3012 Bern (CH)
(72) Inventor: Luu-Dinh, Angélique, 68100 Mulhouse (FR); Zubak, Irena, 3012 Bern (CH)
(74) Representative: Bovard SA Neuchâtel

(57) **Abstract**

The invention relates to a medical device (1) for use in surgery comprising at least one structured substrate (2) comprising a plurality of structures (10), at least a portion of the plurality of structures (10) being a plurality of ridges (101-106, 121-127) having each a length (L) that is at least 5 times greater than their width (W). The ridges (101-106, 121-127) have at least two different cross-sections (C1-C19) and have swellable properties, for example in presence of water.

The medical device (1) comprises at least a second substrate (4), comprising a second plurality of structures (12), said first and second plurality of structures (10, 12) being arranged to interlock when said first substrate (2) is pressed against said second substrate (4) in presence of water.

## Description

### Technical Field

The invention relates to the field of surgical practices and in particular complex dural reconstruction and to the texturization of coatings capable to ensure a tight and hermetic cohesion of the dural substitutes and improve watertight dural closure. More precisely the invention relates to a medical device having two interlockable elements comprising textured coatings.

### Background of the art

In brain or spinal cord surgery the dura mater has to be opened. At the end of the procedure the dura is closed using different techniques depending on the anatomical localization. But when suture of the edges of the remaining dura is not possible, the defect is usually closed using the inlay-overlay technique where one layer of dural substitute is placed beneath and one above the dural defect.

For cerebral and spinal cord surgery, repair of the dura mater can present technical challenges. In particular, insufficient closure of complex dural defects commonly results in cerebrospinal fluid (CSF) leakage This can lead to complications such as meningitis and may require revision surgeries.Multiple options exist for the repair of a dural defect such as surgical sutures with or without application of sealants/adhesives, duroplasty with autologous pericrranium or non-autologous dural substitutes, which are usually made of highly processed equine or bovine collagen. More recently, entirely synthetic and absorbable dural substitutes have received Food and Drug Administration (FDA) approval.This new class of products has the advantage of reduced cortical adhesion and decreased inflammatory reactions compared to biologic materials.. However, for complex dural reconstructions where suturing or application of sealants is impossible the available dural substitutes often dislocate. To overcome the dislocation of the patches there are inventions that engineered mechanical or chemical adhesive solutions to provide more adherence.

For example, US14092015 adhesive articles containing a combination of surface micropatterning such as micro-protrusions and a coating of adhesive glue and methods of making and using thereof. The articles proposed herein exhibit a 90° pull off adhesion of about 1.5 N/cm². The microtopography described doesn't improve substantially the adhesion strength compared to article without microtopography but intend to reduce the quantity of adhesive glue coated and decrease the toxicity of the article.

In KR101867058B, a device is described using hydrogel and manufacturing method of the same. The device describes two surfaces made of hydrogel and containing complementary protrusions arranged in regular arrays. The two surfaces are pressed together such as the protrusions interlock. The interconnected protrusions exhibit a strong and reversible interlocking adhesion in wet or underwater conditions based on the hydration-triggered swelling behavior of the hydrogel polymer. Adhesion for of 80N/cm2 in shear force and 14N/cm2 in pull force are reported after prolonged immersion for 20h in water. This concept may be efficient, however the very tiny dimensions of the protrusions array and the prolonged time to achieve a good adhesion make the method practicably impossible to implement in surgical practices.

### Summary of the invention

It is an objective of the invention to provide a medical device to be used in surgical practices, more precisely in dural reconstruction. The device proposes solution to limitations of solutions of prior art.

The invention proposes a method providing a solution that can be implemented on existing dural substitute consisting in a simple texturization of a swellable hydrogel-based material already Food and Drug Administration (FDA) approved which is improving shelf bonding strength and avoid dislocation of the dural substitute that may also serve as a platform technology in other surgical disciplines.

More precisely the invention relates to a medical device for use in surgery comprising at least a first substrate and at least a second substrate, said at least first substrate comprising a first surface, comprising a first plurality of structures , said at least second substrate comprising a second surface, comprising a second plurality of structures, said first and second plurality of structures being arranged to interlock when said first surface is pressed against said second surface,

At least a portion of said first plurality of structures and/or at least a portion of said second plurality of structures is a plurality of ridges having each a length that is at least 5 times greater than their width, said width and length being defined in a plane parallel to said first and/or second surface. The ridges have at least two different cross-sections defined in planes parallel to said first or respectively said second surface. Said first and second surface may be curved surfaces. The ridges have swellable properties in presence of a wet environment. The swellable properties ensures a better adhesion and/or hermeticity and avoid fluid leakage.

Advantageously a wet environment may be a natural wet environment such as the presence of cerebrospinal fluid (CSF), blood, water or humidity at the place of the closure to be realized. The wet environment may also be enhanced locally by applying a flow of a liquid, such as water, physiological saline solutions, or other solutions used as irrigation fluids during neurosurgical procedure. Such solutions can contain pharmaceutical agents as well as other additives such as tissue healing promoting agents, cell adhesion promoter and the likes.

In order to obtain a strong adhesion faster, and to obtain a better initial hermiticity, other liquids, ions and/or vapors may be used to increase the swelling kinetics of the swellable material of the ridges. For example, physiological saline and other solutions used as irrigation fluids during neurosurgical procedure may be used.

The proposed device of the invention is more efficient, practical and reliable than prior art solutions. Furthermore, the proposed device may be realized by a high productivity process as the swellable structured layer may be tailored during the coating step of the device manufacturing. Providing elongated ridges having said swellable properties according to the invention allows to provide that have a higher adhesion between the two structured substrates than what can be achieved by devices of prior art that only propose arrays of protrusions that have very tiny dimensions. Furthermore, the device of the invention having elongated ridges with the described swelling properties allow to achieve reduced time to achieve a good adhesion, an easier assembly procedure and a less demanding alignment. This makes the device and its method of use much more practical in surgical practices compared to methods and devices proposed in the prior art. Preferably, the device of the invention relies on the use of bio-absorbable and/or degradable materials.

In an embodiment said plurality of ridges are linear-shaped ridges arranged according to concentric squares or rectangles. Adapting the shape of the arrangement of ridges allows to adapt the form in function of the apertures that have to be closed and sealed.

In an embodiment said plurality of ridges are curved-shaped ridges. Curved shaped ridges can be more tolerant to alignment issues as well as deformation and folding of the substrates during surgery procedure. Curved shapes having continuous curvature and no ridges can also provide a better hermeticity in these cases.

In an embodiment said curved-shaped ridges are arranged according to concentric circles or ellipses. Arranging ridges in concentric circles or ellipses can provide an optimum structuration of the portion of the surface interlocking when pressed to each other when closing respectively circular and elliptical apertures.

A concentric arrangement of the interlock elements increases the area of the interlocking element allows to facilitate the manipulation during the operation and ease the alignment and clipping of the two structured layers. In particular, the two substrates can interlock to each other if their centers are facing each other whatever their in-plane orientation is.

In another advantageous embodiment said at least one of said curved-shaped ridges are arranged according to a spiral shape. Using spiral shaped arrangement allows to adapt the attachment and/or peeling force gradually from the center to the border of the arrangement of ridges. It allows to interlock the ridges facing each other by applying a local pressure in a continuous spiral movement, starting from each center and going outwards or the opposite.

In an embodiment said at least two of said plurality of ridges arranged according to different arrangements, preferably a rectangular or square arrangement and a circular or spiral arrangements, or may have a polynomial shape.

In an embodiment said at least a portion of said first plurality of structures and/or at least a portion of said second plurality of structures have different swellable properties. This enables having different adhesion and/or adhesion kinetics in different areas of the devices. This is useful to position the medical device and interlock it partially in a first phase in first areas, to eventually adjust its position before continuing interlocking the two surfaces in other areas. Swelling degree depends on cross-linking degree of the hydrogel. Nature of hydrogel used, the photo-initiator, and the curing time can modify the swelling degree.

In an embodiment said at least a portion of said plurality of ridges have non-uniform swellable properties. Using non-uniform ridges allows to have a higher design flexibility in function of the required attachment and/or peeling forces. This can be realized by providing different level of cross-linking of the swellable materials in different areas, for example using different concentrations of the swellable materials constituent or different cross-linking energy in different areas for example by using ultraviolet light or infrared/heat.

In an embodiment said swellable properties consist in a swelling in the presence of humidity (liquid water or water containing fluids) and wherein said plurality of ridges present a difference in the Young modules of more than 5%, preferably more than 10%, even more preferably more than 20%.

In an embodiment said plurality of ridges present a difference in change of volume between a dry state and a swollen state of more than 5%, preferably more than 10%, even more preferably more than 20%. The change of volume is mostly due to the change in the lateral dimension of the ridges.

In an embodiment the number of said first and/or said second structures is greater than 5, preferably greater than 20, even more preferably greater than 100, possibly greater than 200 .

In an embodiment said wherein the maximal width of the ridges, defined relative to said first or said second planes is smaller than 1000µm, preferably smaller than 500 µm, more preferably smaller than 300µm, even more preferably smaller than 200µm.

In embodiments , the cross sections of said ridges, defined orthogonal to said substrates, may have different shapes chosen among:
- straight-shaped cross-sections
- curved-shaped cross-sections,
- conical cross-sections,
- cross sections presenting overhangs that may have any detailed shape of the overhang part.

In an embodiment, the cross-section of said ridges is homogeneous over the length of the ridges, with less than 10% variation in the profile geometry over portions of said ridges.

In an embodiment, the overlapping width, defined in said first and said second plane, of said first structures with said second structures of said second substrates it is facing, when pressed in contact is smaller than 50µm, or smaller than 30 µm, or smaller than 20 µm, more preferably smaller than 15 µm, or smaller than 10µm, or more preferably smaller than 5 µm. Said overlapping width is defined prior to the swelling

In an embodiment said the peel off force between interlocked said first and said second substrates is higher than 10N/cm², preferably after a swelling time of less than 2 hours.

In an embodiment said first plurality of structures and/or said second plurality of structures comprises at least two alignment structures for aligning said first and second substrates. At least one of the alignment structures may be an aperture.

In an embodiment said first plurality of structures and/or said second plurality of structures comprises at least two conical-shaped apertures for guiding said two alignment protrusions.

### Brief description of the drawings

Further details of the invention will appear more clearly upon reading the following description in reference to the appended figures;
Figure 1 shows a perspective view of a device of the invention comprising ridges that have a length L at least 5 time greater that their maximal width;
Figure 2 illustrates a vertical cross section of two pluralities of interlocking ridges before swelling;
Figure 3 illustrates a vertical cross-section and a top view of two pluralities of interlocking ridges after swelling. The top view illustrates the projected overlapping areas projected in a plane of the device in its interlocked position;
Figure 4 illustrates a perspective vertical cross section illustration two pluralities of interlocking ridges with a portion of the ridges in the interlocked position;
Figure 5 illustrates a perspective vertical cross section illustrating two pairs of substrates with pluralities of interlocking ridges. The figures illustrate a situation with a portion of the ridges in the interlocked position and partially connected by a central structural element;
Figure 6 illustrates a mechanical assembly arranged to peel of an interlocked device from its closed position;
Figure 7 illustrates a top view on a plurality of ridges of a device of the invention;
Figure 8 illustrates a top view on complementary plurality of ridges on a second substrate, to be locked to the plurality of ridges of Figure 6;
Figure 9 illustrates a gap between two opposing ridges before their swelling;
Figure 10 illustrates, horizontal cross sections of the complementary ridges of Figure 6 and Figure 7 in their locked-in position, i.e. when the device is in its closed position, with in bold the overlap of the complementary ridges;
Figures 11 to 20 illustrate embodiments of vertical lateral cross sections of ridges of the device of the invention with varying profiles;
Figure 21 and 22 illustrate a realized ridge of a device of the invention, wherein the ridge presents an overhang realized by micro structuring techniques;
Figure 23 illustrates a picture of a realized part of a device of the invention comprising concentric circular shaped ridges;
Figure 24 illustrates a spiral shaped ridge;
Figure 25 illustrates a plurality of ridges arranged according to centered squares;
Figure 26 illustrates a plurality of ridges arranged according to centered ellipses;
Figure 27 illustrates a vertical cross-section of the process flow of the manufacturing of one substrate containing a single ridge of the medical device.
Figure 28 illustrates a peel off force measurement of an interlocked device after swelling.

### Detailed description and embodiments of the invention

The present invention will be described with respect to particular embodiments and with reference to certain drawings, but the invention is not limited thereto. The drawings described are only schematic and are non-limiting. In the drawings, the size of some of the elements may be exaggerated and not drawn on scale for illustrative purposes. The dimensions and the relative dimensions do not correspond to actual reductions to the practice of the invention.

It is to be noticed that the term "comprising" in the description and the claims should not be interpreted as being restricted to the means listed thereafter, i.e. it does not exclude other elements. Also, by "about" or "approximately" in relation to a given numerical value, it is meant to include numerical values within 10% of the specified value. All values given in the present disclosure are to be understood to be complemented by the word "about", unless it is clear to the contrary from the context.

The indefinite article "a" or "an" does not exclude a plurality, thus should be treated broadly.

Unless defined otherwise, technical and scientific terms used herein have the same meaning as commonly understood by one of ordinary skill in the art to which this invention belongs.

As used herein, the term "compound" referred to substance e.g. biological substance that is composed of one or more materials. In other words, it refers to one or more ingredients which made up a composition. The term "component" as used herein referred to element which is made up of one or several parts e.g. mechanical parts. In other words, the component can be a part that combines with other parts to be functioned or to form something bigger, or to render a specific function when work together with other components.

Reference throughout the specification to "an embodiment" means that a particular feature, structure or characteristic described in relation with the embodiment is included in at least one embodiment of the invention. Thus, appearances of the wording "in an embodiment" or, "in a variant", in various places throughout the description, are not necessarily all referring to the same embodiment, but several. Furthermore, the particular features, structures or characteristics may be combined in any suitable manner, as would be apparent to a skilled person from this disclosure, in one or more embodiments. Similarly, various features of the invention are sometimes grouped together in a single embodiment, figure or description, for the purpose of making the disclosure easier to read and improving the understanding of one or more of the various inventive aspects. Furthermore, while some embodiments described hereafter include some, but not other features included in other embodiments, combinations of features if different embodiments are meant to be within the scope of the invention, and from different embodiments. For example, any of the claimed embodiments can be used in any combination. It is also understood that the invention may be practiced without some of the numerous specific details set forth. In other instances, not all structures are shown in detail in order not to obscure an understanding of the description and/or the figures.

The invention is mainly, but not exclusively related to chirurgical devices.

As used herein, the term "hydrogel material" refers to a material with 3-dimensional structure that can swell in water and hold a large amount of water while maintaining his structural integrity.

The term "overhang" is defined broadly as any structure or layer that is wider than its support portion 5, for example a "T-shaped" structure or the like.

The invention consists of new medical devices with textured surface as dural substitutes to get self-sticking property that provides strong adhesion by means of interlocking of two pluralities of structure made of a material with swellable property, so that they stick firmly together when interlocked and following a swelling. used in the inlay overlay technique

The hydrogel-based material is tailored during the coating step with interlock geometrical elements, especially tailored by structuring its layer in specific 3-dimensional shapes. The specific overhang shape of the structured swellable material in two pluralities of ridges on two complementary substrates is engineered such as to interlock the two structured layers in dry environment and ensure a hermetic bounding through the water-swollen property of the hydrogel material in wet or under-water environment. Preferably the overhang shape is a "T-shape".

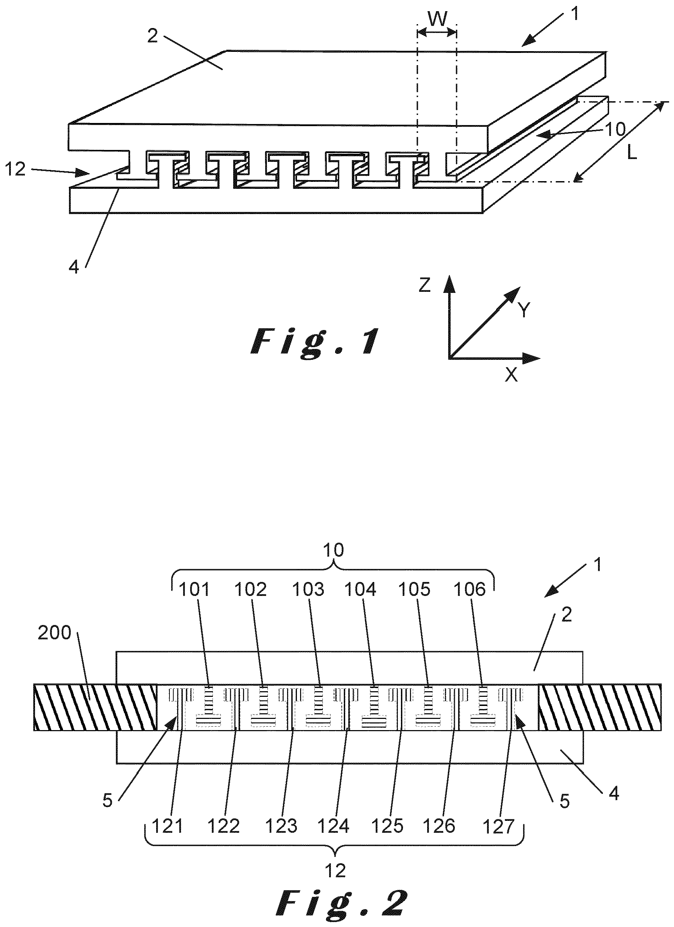

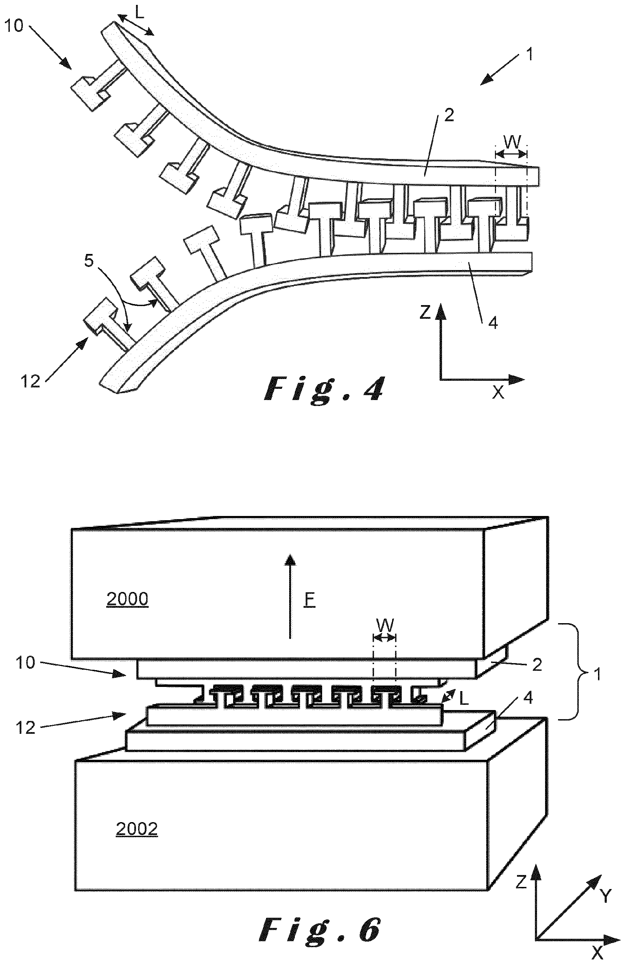

More precisely the invention relates to a medical device 1 for use in surgery comprising at least a first substrate and at least a second substrate, said at least first substrate comprising a first surface, comprising a first plurality of structures , said at least second substrate comprising a second surface, comprising a second plurality of structures, said first and second plurality of structures being arranged to interlock when said first surface is pressed against said second surface and after the swelling of at least one of said first or second plurality of structures, as illustrated in Figure 1 and 4. Swelling is defined by the swelling ration SR: SR= (M_{hydrated}-M_{dehydrated})/M_{dehydrated} Herein, M means the mass of the structures that undergo swelling.

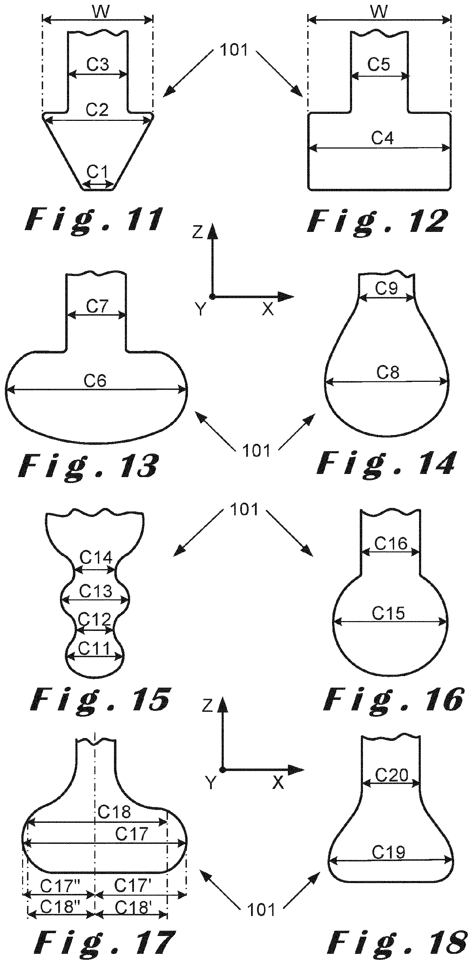

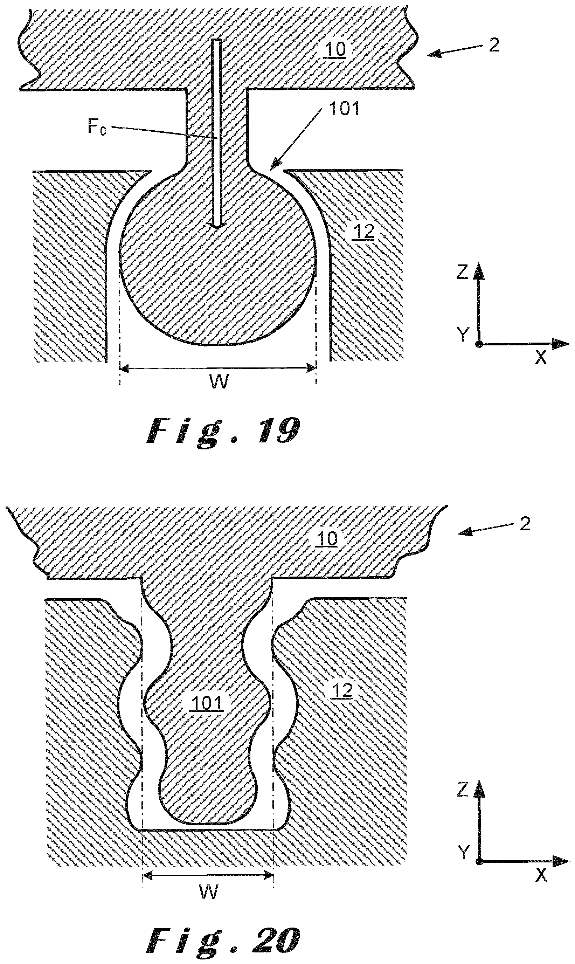

At least a portion of said first plurality of structures 101-106 and/or at least a portion one of said second plurality of ridges 121-127 is a plurality of ridges having each a length L that is at least 5 times greater than their width W, said width W being defined in a plane parallel to said first and/or second surface, as illustrated in Figure 4-6 It is understood that in some structures, such as the ones illustrated in Fig.15 or 20 the structures may have different sections having different widths In such cases the width W is referred as the maximal width of the structure, for example the width of section C13 in Fig.15

Said first plurality of ridges 101-106 and/or at least one of said second plurality of ridges 121-127 have swellable properties in presence of a liquid and/or vapor. In an embodiment a gas may be introduced in the liquid to modify the swelling kinetics or swelling ratio, or to improve the substrate adhesive properties

In embodiments the swelling of the ridges 101-106, 121-127 may be realized or accelerated by varying the pH, and/or the temperature. For example, for a pH greater than 10 a swelling of more than 200% in volume may be obtained. It is referred here to the following publication: https://akinainc.com/polyscitech/products/aquagel/AquaGel-pH.php

In an embodiment said at least one of said first plurality of structures 101-106 and/or at least one of said second plurality of ridges 121-127 have at least two different cross-sections (C1-C20) defined in at least two virtual surfaces parallel to said first, respectively said second surfaces.

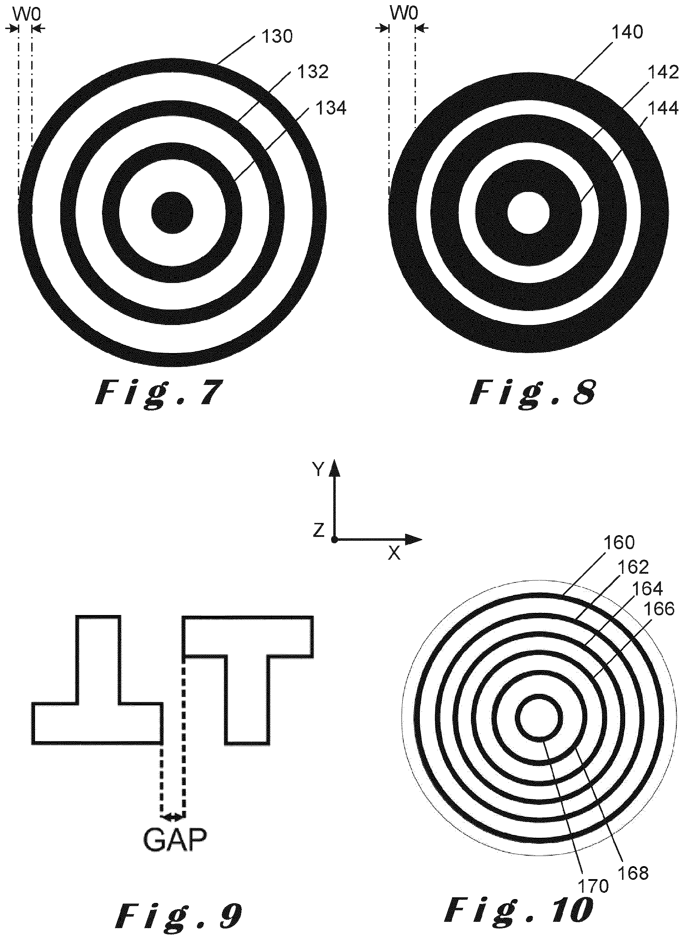

In embodiments, illustrated in the Figures 11-20 , the cross sections, defined orthogonal to said substrates, may have different shapes chosen among:
- straight-shaped cross-section (Figures 9, 12)
- curved-shaped cross-sections (Figures 13, 14, 16-20),
- conical cross-section, (Figure 11),
- cross sections presenting overhangs that may have an overhang part comprising different sags (Figures 15, 20),
- cross section may be asymmetrical comprising different lateral radii (C17', C17", C18', C18") as illustrated in Figure 17.

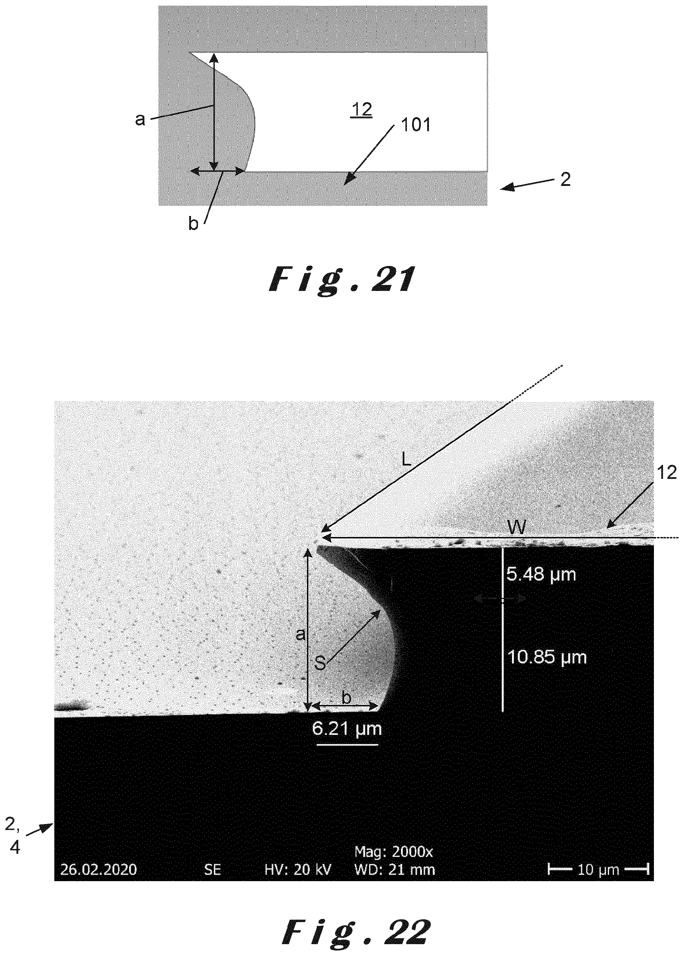

Fig.21 and Fig.22 shows a typical lateral cross section of a ridge 101 having a height a and an overhang b. Fig.22 shows a realized ridge wherein the structure has a first curved section having a height of 10.85 µm and a second curved section having a height of 5.48 µm, the overhang width b being about 6.21 µm. The details shape S of the cross section of an overhang structure, as illustrated in Fig.21 and 22 may be defined by a polynomial. In an embodiment, said plurality of ridges are linear-shaped ridges arranged according to concentric squares or rectangles.

In an embodiment said plurality of ridges are curved-shaped ridges 101-106 , 121-127.

In an embodiment said curved-shaped ridges 101-106 , 121-127 are arranged according to concentric circles or ellipses.

Preferably, at least of said first and second substrates 2, 4 are flexible foils, preferably thin, with a thickness below 1mm, and are preferably at least partially stretchable. As examples of substrate materials, organic materials such as polymer containing biocompatible materials are preferred. As examples, graft materials made of bovine or equine collagen sources, or synthetic material such as Polyethylene terephthalate (PET), Polyurethane (PU), and more preferably synthetic and resorbable such as Polylactic acid (PLA), Polyglycolide (PGA), poly-caprolactone (PLC) can be used.

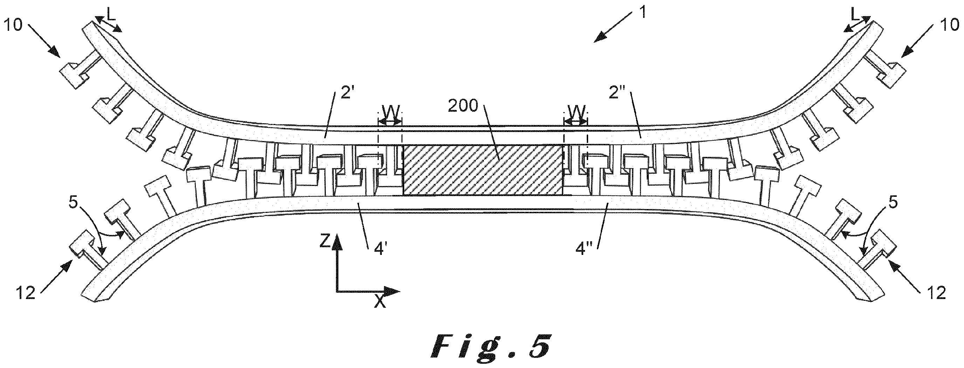

In embodiments the medical device 1 comprises more than one interlockable substrates as illustrated in Fig.5. Fig.5 illustrates a variant in which two pairs of opposite substrates 2',4', 2", 4" are connected by a central structural element 200. Such structural element 200 is preferable a flexible element that may be configured as a pivoting axis such as a flexible layer extending over at least a portion of a length of the substrates 2',4', 2", 4". In variants the structural element 200 may be connected to all the substrates 2',4', 2", 4", or to two of them. For example, the structural element 200 may be a flexible mechanical connection between two first substrates 2, 2' or between two second substrates 4', 4" or between a first substrate 2', 2" and an opposite second substrate 4, 4'.

In variants, not illustrated, a plurality of first and second substrates may be assembled and interconnected by pivoting axes or flexible elements or bridges so that the medical device can be applied onto a round surface to be covered and healed.

It is understood that first and second substrates may have any shape, such as a circular, elliptical or rectangular shape.

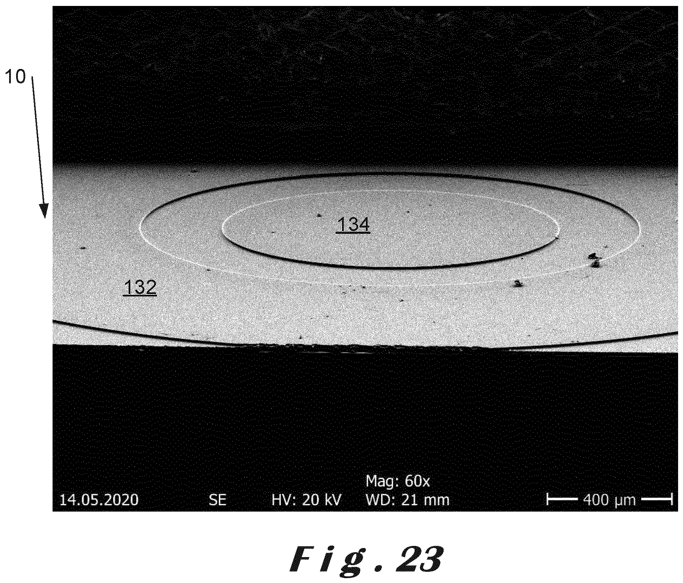

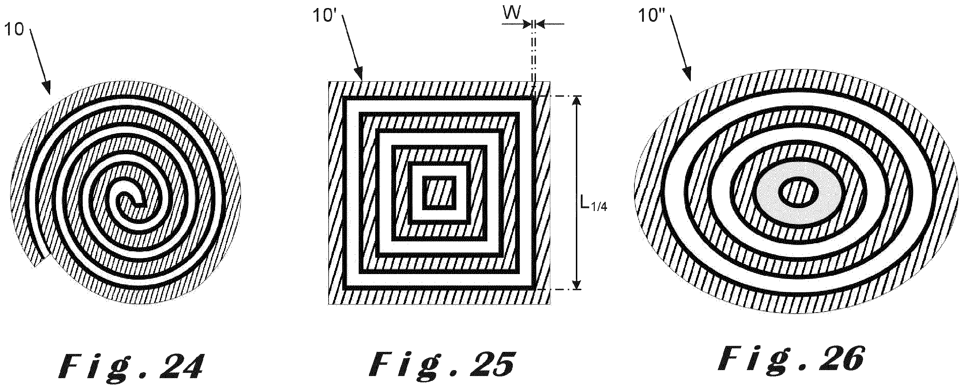

To increase the area of bonding, the interlock element of the invention may be engineered and organized in concentric arrangement with a simple axis of rotation to facilitate the manipulation during the operation and ease the alignment and clipping of the two layers. This is illustrated in Figures 23, 26.

In another advantageous embodiment said at least one of said curved-shaped ridges are arranged according to a spiral shape (Figure 24).

In an embodiment said at least two of said plurality of ridges arranged according to different arrangements, preferably a rectangular or square arrangement and a circular or spiral arrangement.

Figure 7 illustrates a top view on a first concentric arrangement of circular-shaped ridges 130, 132, 134 of a device 1 of the invention

Figure 8 illustrates a top view on a second concentric arrangement of circular-shaped ridges 140, 142, 144 on a second substrate, to be locked to the plurality of ridges 130, 132, 134 of Figure 7.

In exemplary executions the concentric ridges of the embodiment of Figure 7 and Figure 8 may have the following (table 1) combination of typical dimensions of widths of the ridges and their projected gaps. The gaps are defined as the projected aperture between opposing structures before their assembly and swelling so that the stick together after swelling.

**Table 1: Typical widths of a first 101-106 and second 121-127 array of concentric ring ridges and the typical gap between them after assembly.**

| First concentric ridge width W[microns] | Second ridge width W[microns] | Gap between first and of second concentric ridges [microns] |
|---|---|---|
| | | |
| 150 | 250 | 0 |
| 150 | 250 | ±5 |
| 150 | 250 | ±10 |
| 550 | 1050 | 0 |
| 550 | 1050 | ±5 |
| 550 | 1050 | ±10 |

The ridges comprise preferably a base 5, illustrated in Figures 2,5 that may have a height, defined orthogonal to its local support area, that is greater or smaller or equal than said with W. In an embodiment said at least a portion of said first plurality of ridges 101-106 and/or at least a portion of said second plurality of ridges121-127 have different swellable properties.

In an embodiment said at least a portion of said plurality of ridges 101-106 ,121-127 have non-uniform swellable properties.

In an embodiment said swellable properties consist in a swelling in the presence of humidity and wherein said plurality of ridges 101-106 ,121-127, present a difference in the Young modules of more than 5%, preferably more than 10%, even more preferably more than 20%.

In an embodiment said plurality of ridges 101-106 ,121-127 present a difference in elongation between a dry state and a swollen state of more than 5%, preferably more than 10%, even more preferably more than 20%.

In an embodiment said the number of said first and/or said second structures is greater than 5, preferably greater than 20, even more preferably greater than 100 or even greater than 200.

In an embodiment said wherein the maximal width of said plurality of ridges 101-106,121-127, defined relative to said first or said second planes is smaller than 1000µm, preferably smaller than 500 µm, more preferably smaller than 300µm.

In an embodiment said the overlapping width W0, defined in said first and said second plane, of said first structures and/or said second structures when pressed in contact is smaller than 50µm, preferably smaller than 30 µm, more preferably smaller than 15 µm, even more preferably smaller than 10 µm, even more preferably smaller than 5 µm.

In an embodiment the peel off force between interlocked said first and said second substrates is higher than 10N/cm².

In embodiments the adhesion between the structures may be improved by a coating chosen among: collagen, fibrin, gelatin or a combination of such coatings.

In an embodiment said first plurality of ridges 101-106 and/or said second plurality of ridges ,121-127 comprises at least two alignment protrusions for aligning said first and second substrates.

In an embodiment said first plurality of ridges 101-106 and/or said second plurality of ridges 121-127 comprises at least two conical-shaped apertures for guiding said two alignment protrusions.

In a second aspect the invention relates to a method of fabrication of the medical device 1.

To define the optimized shape of the medical device 1, the selection is based by design iterations essentially considering manufacturing limitation aspects such as the aspect ratio (width versus height of the structure) undercut size to ensure good molding without structure breaks during the demolding phases. An optimal bounding area and the mechanical and swelling behavior of the hydrogel are taken in consideration.

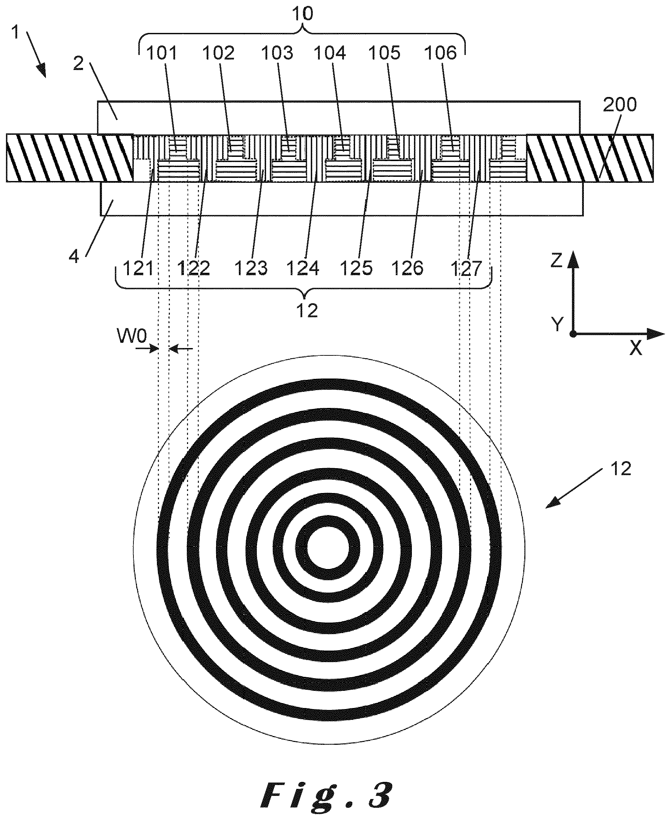

For example, based on iterative manufacturing trials the shape of an advantageous geometry of the medical device 1 is fixed as illustrated in the Figure 3:
- the cross-section of the interlocking element 101-106 , 121-127 has a height of 15 microns,
- the cross-section of the interlocking element 101-106 , 121-127 has an overhanging width of 5-7 microns

Simulation of the performance of the interlocking features 101-106 , 121-127 have been performed by finite element analysis with the software Comsol Multiphysics.

For the theoretical model a mesh with node elements was designed. The geometry was constructed based on the manufactured sample architecture. The material used for the study is a photo-curable Poly(ethylene glycol) diacrylate (PEGDMA). Table 2 show the material properties (it is referred here to the publication "Flexible and Shape-Reconfigurable Hydrogel Interlocking Adhesives for High Adhesion in Wet Environments Based on Anisotropic Swelling of Hydrogel Microstructures", by Hyun-Ha Park and all, https://doi.org/10.1021/acsmacrolett.7b00829) used as input parameters.

**Table 2: Elastic moduli and elongation at break of the hydrogel material in dry and swollen state**

| | Dry state | Swollen state |
|---|---|---|
| Young's modulus (MPa) | 61.7 ± 6.5 | 60.3 ± 6.5 |
| Elongation at break (%) | 10.2 ± 6.5 | 5.1 ± 0.6 |

### Interlocking geometrical element fabrication

For the experiment, PEGDMA (molecular weight 550) polymer and 0.5% of 2-hydroxy-2-methylpropiophenone photo-initiator were mixed before used. The polymeric negative tone resist (ma-N-440, Microresist) and lift-off resist LOR30B, Microresist) were used as received.

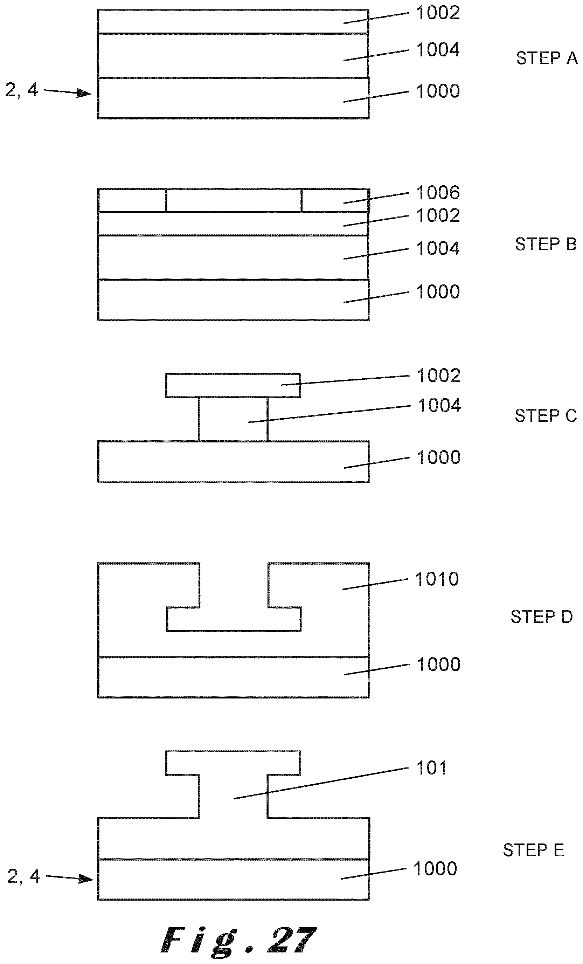

The required interlocking geometrical elements was manufactured following different fabrication steps wherein a template matrix is fabricated by photolithography. This is illustrated in Figure 27

The main process steps are the following:
**Step A:** fabricate a template matrix comprising the steps of:
   - spin-coating two layers 1002, 1004 of photoresists on a glass substrate 1000, where a bottom layer of lift-off resist is a polymeric based on polymethylglutarimide (PMGI) 1004 having a high developability, and a top layer of polymeric negative tone photoresist 1002 sensitive in the range of the i-line (360nm);
   - baking of the PMGI layer 1004 alone and then with the top layer 1002 by way of using a hotplate;
**STEP B** :patterning the layer
   - using contact photolithography with a chromium photomask (p) to expose the pattern on the top layer 1002;
**STEP C** remove photoresist, consisting in:
   - using a developer to develop and remove selectively the layer 1002 and 1004 to form the "T" shape.
**STEP D** realizing an overhang structure mold, consisting in :
   - after origination of the targeted shape such as a "T" shape, a flexible polydimethylsiloxane (PDMS) mold 1010 having a configuration opposite to that of the template matrix is realized by pouring a precise amount of the PDMS mixture on the photoresist matrix to obtain a desired thickness;
   - curing of the mold 1010 material by heating at a precise temperature and time
   - separation of the mold 1010 from the layer 1002-1004 template.
**STEP E** realizing the ridges 101-106, 121-127, by:
   - obtaining a PEGDMA coating 101 by dispensing the precise amount of material between the dural substitute and the PDMS mold to obtain the desired thickness;
   - crosslinking of the material and separation of the cured PEGDMA patterned layer from the mold 1010.
This provides the first substrate or the second substrate, and the operation is repeated with a complementary layout to provide the other substrate. Alternatively, one can provide both the first and second substrates after cutting a larger foil in which layout are incorporated in parallel both first and second substrate layouts.

### Alignment features

The interlock element 1 is preferably organized in concentric arrangement. Each array of concentric circles has an interlock array structure having the opposite configuration. For the experimental study circles with different widths W and gap tolerance were evaluated and are summarized in the Table 2 (further paragraph experimental results).

Additional alignment helping features can be present on the medical devices 1 . For example, printed moire or Vernier pattern on said first and said second substrate can provide a visual help to perform the alignment of both substrates. For example, bio-absorbable blue ink concentric complementary circle on both substrates can provide a visual guidance of the alignment process.

Additionally, other printed patterns can interfere and provide a distinctive pattern, for example a moire pattern, when in close contact. This can provide a visual indication of having the first and second substrates in contact and there at least partially locked.

Other visual non printed features can be implemented on the two substrates 2, 4 to provide indications of alignments and proximity of the two substrates 2,4. As example, micro or nanostructure can provide variable optical effect related to the alignment and proximity of structures located on both substrates. Other features can be implemented to help alignment with imaging tools in the visible, infrared, terahertz spectra or with ultrasonic imaging. Parts of the two substrates 2, 4 can be arranged to have a high transparency to help alignment processes.

### Experimental results:

Devices with different concentric arrangements were realized by using the method described above and applied on a 25mm² PET foil carrier in place of the dural substitute. Figure 23 shows a top view of 2 ridges 132, 134 of a realized substrate comprising a plurality 10 of ridges.

Values of realized samples are summarized in Table 2. Table 2 shows the values that have been obtained by experimental optimization that consisted in varying the ring dimensions such as their position and ridge widths and dimension and shape of the ridge edge as illustrated on Figures 21 and 22.

**Table 2: this table summarizes geometries that have provided the best results. They are based on a concentric arrangement of the ridges that have a thickness of 150 µm for the structures on a first substrate and 450µm for the rings of the structures on the second substrate 4.**

| | Common for all options | Option A | Option B | Option C |
|---|---|---|---|---|
| Ridge number | Ridge width (W) [microns] | Ridge's edge from center [microns] no gap (in case of perfect alignment) | Ridge's edge from center [microns] 5 microns gap between two adjacent ridges | Ridge's edge from center [microns] 10 microns gap between two adjacent ridges |
| 0 | - | 500 | 495 | 480 |
| 1 | 150 | 650 | 645 | 630 |
| 2 | 400 | 1050 | 1045 | 1030 |
| 3 | 150 | 1200 | 1195 | 1180 |
| 4 | 400 | 1600 | 1595 | 1580 |
| 5 | 150 | 1750 | 1745 | 1730 |
| 6 | 400 | 2150 | 2145 | 2130 |
| 7 | 150 | 2300 | 2295 | 2280 |
| 8 | 400 | 2700 | 2695 | 2680 |
| 9 | 150 | 2850 | 2845 | 2830 |
| 10 | 400 | 3250 | 3245 | 3230 |
| 11 | 150 | 3400 | 3395 | 3380 |
| 12 | 400 | 3800 | 3795 | 3780 |
| 13 | 150 | 3950 | 3945 | 3930 |
| 14 | 400 | 4350 | 4345 | 4330 |
| 15 | 150 | 4500 | 4495 | 4480 |
| 16 | 400 | 4900 | 4895 | 4880 |
| 17 | 150 | 5050 | 5045 | 5030 |
| 18 | 400 | 5450 | 5445 | 5430 |
| 19 | 150 | 5600 | 5595 | 5580 |
| 20 | 400 | 6000 | 5995 | 5980 |

To examine the interlocking behavior and the performance of the devices, the interlocking shear and normal strength were measured with a tensile tester for pull-off and peel tests in wet condition.

For the experiment, two devices with complementary configuration were put into contact with each and expose to water for one hour before the test.

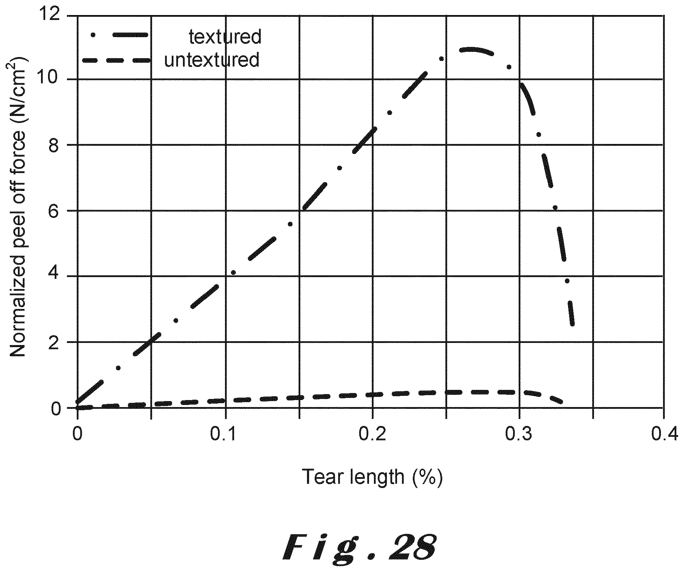

Figure 6 illustrates a mechanical assembly comprising two supports 2000 arranged to measure the peel-off force of an interlocked device 1 from its closed position. Figure 28 illustrates the response of the assembly with the PEGDMA surfaces texturized compared to a reference without surface texturization.

The texturization has demonstrated, the ability to improve by a factor 10 the cohesion of the assembly compared to an untextured one with more than 10N/cm² peel off strength achieved within a short immersion in water (one hour).

### Implementation examples

The combination of interlocking geometry combined with the intrinsic material property will enhance the performance of dural substitute existing products and thus it is intended to significantly decrease cerebrospinal fluid leakage and post-operative complications and avoid revision surgeries.

The concept of modifying surface properties of implantable materials may also serve as a platform technology in other surgical disciplines:
Peritoneal repair surgery (such as hernia repair or any other situation where closure of the abdominal wall is needed), especially during minimally invasive surgery with laparoscopy where surgical suturing is extremely difficult to handle and very time consuming. As the use of minimally invasive surgery is increasing, the need for self-closing devices are gaining a lot of importance.

Pleura repair surgery. Similar to peritoneal repair, minimally invasive surgical techniques such as thoracoscopy are on the rise. After each surgery the pleura needs to be closed in a water- and air-tight manner.

Surgery of the oral or nasal cavity where suturing is impossible, e.g. repair of mucous membranes, repair of the eardrum, reconstruction of the palate.

Vascular surgery: any situation where larger blood vessels need to be closed such as in bypass surgery or vascular reconstruction surgery. Additionally, vascular substitute materials (which are usually made of Gore-Tex) could be structured to enhance tightness of closure.

Every situation where an anatomical cavity consisting of a thin wall of connective tissue needs to be closed tightly. Such situations are found in closure of dura mater, peritoneum or pleura.

Every surgical situation where a thin layer of connective tissue needs to be closed and suturing is not possible or cosmetically not appropriate, e.g. pediatric surgery, plastic surgery.

## Claims

1. A medical device (1) for use in surgery comprising at least a first substrate 2 and at least a second substrate 4, said at least first substrate 2 comprising a first surface, comprising a first plurality of structures (10), said at least second substrate 4 comprising a second surface, comprising a second plurality of structures (12), said first and second plurality of structures (10, 12) being arranged to interlock when said first surface is pressed, by applying a force Fo, against said second surface,
wherein
at least a portion of said first plurality of structures (10) and/or at least a portion of said second plurality of structures (12) is a plurality of ridges (101-106, 121-127) having each a length (L) that is at least 5 times greater than their width (W), the width (W) and the length (L) being defined in a plane parallel to said first and/or second surface,
said ridges (101-106, 121-127) have at least two different cross-sections (C1-C20) defined in planes parallel to said first, respectively said second surface,
said ridges (101-106, 121-127) have swellable properties in presence of a liquid and/or vapor.

2. The medical device according to claim 1 or 2 wherein said plurality of ridges (101-106, 121-127) are linear-shaped ridges arranged according to concentric squares or rectangles.

3. The medical device according to claim 1 wherein said plurality of ridges (101-106, 121-127) are curved-shaped ridges.

4. The medical device according to claim 4 wherein said curved-shaped ridges are arranged according to concentric circles or ellipses.

5. The medical device according to claim 4 wherein at least one of said curved-shaped ridges are arranged according to a spiral shape.

6. The medical device according to any one of claims 1 to 5 wherein at least two of said plurality of ridges (101-106, 121-127) arranged according to different arrangements chosen among: rectangular, square, circular, spiral, polynomial shapes.

7. A medical device according to any one of claims 1 to 6 wherein at least two portions of said ridges (101-106, 121-127) have different swellable properties.

8. A medical device according to any one of claims 1 to 7 wherein at least a portion of said plurality of ridges have non-uniform swellable properties.

9. A medical device according to any one of claims 1 to 8 wherein said swellable properties consist in a swelling in the presence of humidity and wherein said plurality of ridges (101-106, 121-127) present a difference in the Young modules of more than 5%, preferably more than 10%, even more preferably more than 20%.

10. A medical device according to any one of claims 1 to 9 wherein said plurality of ridges (101-106, 121-127) present a difference in elongation between a dry state and a swollen state of more than 5%, preferably more than 10%, even more preferably more than 20%.

11. A medical device according to any one of claims 1 to 10 wherein the number of said ridges (101-106, 121-127) is greater than 5, preferably greater than 20, preferably greater than 100, even more preferably greater than 200.

12. A medical device according to any one of claims 1 to 11 wherein the maximal width (w) of said plurality of ridges (101-106, 121-127) is smaller than 1000µm, preferably smaller than 500 µm, more preferably smaller than 300µm, said widths (w) being defined relative to said first or said second planes.

13. A medical device according to any one of claims 1 to 13 wherein the overlapping width (WO), defined in said first and said second plane, of said ridges (101-106, 121-127), when pressed in contact is smaller than 50µm, preferably smaller than 30 µm, preferably smaller than 20 µm, more preferably smaller than 15 µm, even more preferably smaller than 10 microns, even more preferably smaller than 5 microns.

14. A medical device according to any one of claims 1 to 13 wherein the peel off force F between interlocked said first and said second substrates is higher than 10N/cm².

15. A medical device according to any one of claims 1 to 14 wherein said first plurality of structures and/or said second plurality of structures comprises at least two alignment protrusions for aligning said first and second substrates.

16. A medical device according to claim 15 wherein said first plurality of structures and/or said second plurality of structures comprises at least two conical-shaped apertures for guiding said two alignment protrusions.

17. A medical device according to any one of claims 1 to 16 wherein said at least first substrate 2 and the said at least second substrate 4 are bonded in at least one bond area for aligning said first and second substrates.
